# EUROPEAN PATENT APPLICATION

(11) **EP 3 786 350 A1**
(43) Date of publication of application: **03.03.2021**
(21) Application number: 20189584.4
(22) Date of filing: 05.08.2020
(51) Int. Cl.: D06M 16/00, D06M 13/352

(54) **METHOD FOR THE SANITIZING TREATMENT OF A TEXTILE PRODUCT**

(30) Priority: 26.08.2019 IT 201900015024
(71) Applicant: Manteco S.p.A., 59013 Prato (IT)
(72) Inventor: MANTELLASSI, Matteo, 59100 PRATO (IT); MANTELLASSI, Franco, 59100 PRATO (IT); MANTELLASSI, Marco, 59100 PRATO (IT)
(74) Representative: Firmati, Leonardo

(57) **Abstract**

Described is method for the sanitizing treatment of a textile product comprising a step of providing a textile product, a step of washing said textile product and a subsequent sanitizing treatment step, said sanitizing treatment step comprising the application on said textile product of a mixture based on methylisothiazolinone (MIT) and methylchloroisothiazolinone (CIT) and water.

## Description

This invention relates to a method for the sanitizing treatment of a textile product.

The need is currently increasingly felt to perform sanitizing treatments on the textile products in particular during the first steps of production of the textile product.

The sanitizing of the textile product is fundamental since any proliferation during its production steps of bacteria, algae, fungi or moulds generates drawbacks which are often irreversible on the products.

One of the steps of producing the textile product in which a contamination, for example bacterial, is most likely, is that in which the raw materials are stationary for medium periods of time inside closed storage units. The stores of raw materials are generally closed rooms or buildings, inside of which large changes in temperature and humidity occur during the day and the various seasons.

These conditions, in addition to the fact that the raw materials are organic materials (for example wool, cotton, linen, etc.), means that the proliferation of bacteria, algae, fungi or mould is very frequent.

The main problem which occurs if the raw materials are attacked by bacterial contaminating agents or the like is due to the fact that these attacks are mainly invisible to the naked eye and are only evident in the subsequent production steps.

Merely by way of example, if the raw materials constituting a piece of textile product have undergone a bacterial attack during a first production step (for example, storage in a warehouse, bale opening, flock opening, dusting...) is not for the user to easily and instantaneously identify it.

Generally speaking, a step following which the user is able to make a first identification of any contamination is that following dyeing of the piece of textile product. It is known that if a portion of textile product in production is attacked by bacteria the dyeing step can generate many problems.

A first problem may be the fact that the dyeing in the portion attacked by the bacteria does not fix in a suitable manner to the textile product causing loss of colour in the subsequent steps of production of the textile product or, sometimes, following the first washing performed by the user of the finished garment. The same problem occurs when the piece of textile product is attacked by algae, fungi or mould.

Even if the operator responsible for carrying out an initial check on the quality of the textile product being produced realises that a portion of the piece has been contaminated, the problem is no less important.

In effect, the only solution is to clearly signal, by marking it, the portion in which the dye has not adhered in a suitable manner in such a way as to allow a clear identification to the successive users once the piece has been completed.

In this way, the users of the subsequent steps, until reaching the packaging of the finished garment, will have the possibility of eliminating the portion(s) attacked by contaminating agents to ensure that they are not present in the finished products.

Clearly, this elimination of the contaminated portions considerably increases the waste of textile product, thus considerably increasing the overall production costs and the average pollution generated in the production of textile products.

There are currently no known sanitizing treatment methods in the sector for the production of textile products which resolve the above-mentioned problem.

In effect, currently, the only procedure implemented following an identification of a portion of piece of textile product by an operator is to subject the piece to a series of extra washes.

However, washing the piece often does not solve the problem of the persistence of the contaminating charge (bacteria, fungi, mould) which survives all the washing cycles performed with traditional solvents without particular antibacterial characteristics.

As a result, even a subsequent re-dyeing step does not achieve adequate results, with chromatic and structural uniformity of the piece.

Moreover, a further drawback is caused by the fact that subjecting the contaminated textile product to series of extra washes increases significantly the pollution generated in the production of the textile products.

A first aim of the invention is therefore to provide a method for the sanitizing treatment of a textile product which overcomes the above-mentioned problems and which in particular allows the risk of contamination of a textile product during its production steps to be minimised.

A second aim of the invention is to provide a method for the sanitizing treatment of a textile product which is able to sanitize the entire production process of the textile product, reducing the waste of product and therefore the costs and the pollution of the entire production process.

The above aims are achieved by a method for sanitizing a textile product comprising the technical features described in one or more of the accompanying claims. Further features and advantages of the invention are more apparent in the non-limiting description which follows of a preferred embodiment.

The method for sanitizing a textile product according to the invention comprises in its basic form a first step of providing a predetermined quantity "X" of a textile product, a second step of washing the textile product provided and a third step of sanitizing the textile product.

Advantageously, the sanitizing treatment is performed after the washing step in such a way that it does not reduce, even partly, the sanitizing capacity of the treatment.

The sanitizing treatment step comprises applying on the textile product, already washed, a quantity "Y" of an anti-contaminating mixture based on methylisothiazolinone (MIT) (C₄H₅NOS) and methylchloroisothiazolinone (CIT) (C₄H₄ClNOS) and water. The quantity of anti-contaminating mixture Y is between 0.1% by weight and 1% by weight of the quantity X of textile product to be treated.

Advantageously, the above-mentioned range between Y and X is between 0.3% by weight and 0.4% by weight.

The term "textile product" used in the invention denotes any textile material during both the pre-weaving step (raw materials in the form of fibres or flock) and the subsequent steps of weaving (piece of textile product) up to the finished garment.

This means that the sanitizing treatment method can be performed on any of the above-mentioned stages of textile product during the entire production process of the textile product.

Advantageously, it is clear that the further upstream of the various steps for production of the textile product the product is sanitized there is less risk that there may be proliferation of harmful contaminating agents in the subsequent production steps.

Methylisothiazolinone (MIT) and methylchloroisothiazolinone (CIT) are synthetic anti-microbial and antibacterial substances with respect to both gram-positive and gram-negative bacteria. Advantageously, the anti-contaminating mixture used for the sanitizing treatment step is diluted in water in a percentage of between 3% by weight and 10% by weight. Still more preferably, the percentage is between 6% by weight and 7% by weight.

Advantageously, in order to determine in an optimum manner the various percentages and the ratios between them reference is made to the quantity X of textile product to be treated.

In the case of the dilution in water described above there is therefore a total recovery: anti-contaminating mixture (0.1%-1% by weight of the quantity X of textile product to be treated), water (3%-10% by weight of the quantity X of textile product to be treated). Percentage dilution values may be used as a function of the specific production step of the textile product where the sanitizing treatment occurs.

As mentioned above, advantageously, the step of providing the textile product comprises the step of selecting untreated textile fibres, that is to say, textile fibres in the form of a raw material.

In the case of non-recycled wool the untreated textile fibres are those fibres which come from the shearing of the animals and which after a first washing are stored in respective stores.

In the case, on the other hand, of recycled wool the untreated textile fibres are those fibres which come from the recycling operations of the textiles coming from garments packaged at the end of their life or waste of textiles from the various production steps. Advantageously, the sanitizing step of the method according to the invention is performed as a first working step on the unprocessed fibres, that is to say, the first step after the movement of the fibres from the respective store towards the "opening" operations.

In this way it is possible to achieve a high level of sanitization not only on the unprocessed textile fibres but also on all the textile products which are obtained during the subsequent steps starting from them.

In effect, starting from sanitized raw materials in which every possibility of contaminating proliferation is obstructed, it is clear that all the products obtained from them are in the same way sanitized and have the same anti-contaminating characteristics.

In this way it is possible to avoid the risk of contaminating proliferations (for example, based on bacteria, fungi, algae or mould) during all the steps of producing a textile product until packaging the finished garment.

In a preferred embodiment of the invention, the sanitizing treatment step is performed together and/or simultaneously with a step of sizing the textile product. The sizing step is a production step consisting of the oiling of the textile mix which is used to increase the inter-fibrous friction coefficient, which favours the cohesion of the card web and of the threads.

At the same time, the sizing allows the friction between the textile product being produced and the metal seals of the machines to be reduced.

The substances used in the traditional sizing step (that is to say, without the sanitizing treatment step according to the invention) generally comprise a mixture of oil emulsifiers, emulsifiers, softeners, additives, cohesion agents, condensates, antistatic agents and water.

More specifically, therefore, in the corresponding sizing machine, the mixture of textile product is lubricated or, rather, added to, with one or more of the above-mentioned components mixed with the anti-contaminating mixture based on methylisothiazolinone (MIT) and methylchloroisothiazolinone (CIT) according to the invention.

If the sanitizing treatment step is performed during the sizing step, the anti-contaminating mixture based on methylisothiazolinone (MIT) and methylchloroisothiazolinone (CIT), starting, for example, from 1000 kg of textile product to be treated (quantity X), we will have:
1-10 kg of anti-contaminating mixture,
6-7 kg of water,
7-8 kg of oil emulsifiers,
2-3 kg of antistatic agent.

Advantageously, if the sanitizing treatment is performed during the step of sizing the textile fibres, it may be actuated by independent spraying means for the anti-contaminating mixture with respect to the spraying means for the traditional lubricating mixture.

The sanitizing treatment method according to the invention may be actuated on any type of textile product such as, for example, new wool, recycled wool, cotton, linen, hemp, silk.

Advantageously, the sanitizing treatment step according to the invention is performed by means of an operation for spraying the anti-contaminating mixture based on methylisothiazolinone (MIT) and methylchloroisothiazolinone (CIT) on the textile product, whether it is in the form of fibre, flock or piece.

In a further solution, the step of sanitizing the textile product comprises a step of immersion of the textile product in the anti-contaminating mixture based on methylisothiazolinone (MIT) and methylchloroisothiazolinone (CIT) in a suitable tank or container.

In order to obtain a greater impregnation and therefore overall effectiveness of the textile product with the anti-contaminating mixture based on methylisothiazolinone (MIT) and methylchloroisothiazolinone (CIT) the above-mentioned immersion step (designed to perform the sanitizing step) also comprises a step of mechanical stirring of the textile product.

Advantageously, the anti-contaminating mixture based on methylisothiazolinone (MIT) and methylchloroisothiazolinone (CIT) has a density at 20° of between 1.017 and 1.037 g/cm³, a pH value at 20° of between 3.0 and 4.0 and a refraction index at 20° of between 1.3380 and 1.3440.

The invention achieves the preset aims, overcoming the drawbacks of the prior art.

More specifically, the method according to the invention makes it possible to obtain in any step of production of a textile product the elimination of any contaminating proliferations such as, for example, bacteria, algae, fungi, mould.

The above-mentioned advantage is obtained from the introduction in the production process of the MIT and CIT-based anti-contaminating mixture which carry out their powerful anti-microbial and anti-bacterial action. In short, a textile product produced from textiles treated according to the method of the invention is a textile which is particularly sanitized and free of contaminating charges (such as, for example, bacteria, fungi, algae and mould) which can adversely affect the chromatic or structural stability of the fabric.

## Claims

1. A method for the sanitizing treatment of a textile product comprising the following steps performed in succession:
- providing a predetermined quantity (X) of textile product,
- washing said textile product and
- sanitizing said washed textile product, said sanitizing step comprising the application on said textile product washed of a quantity (Y) of an anti-contaminating mixture based on methylisothiazolinone (MIT) and methylchloroisothiazolinone (CIT) and water, wherein the quantity of mixture (Y) is between 0.1% by weight and 1% by weight of said quantity of textile product (X).

2. The method according to claim 1, wherein said anti-contaminating mixture is diluted in said water in a percentage of between 3% by weight and 10% by weight.

3. The method according to any one of the preceding claims, wherein said step of providing said textile product comprises the step of selecting unprocessed textile fibres.

4. The method according to any one of the preceding claims, wherein said step of sanitizing treatment is carried out together with a step of sizing said textile product.

5. The method according to claim 4, wherein said sizing step comprises applying on said textile product said anti-contaminating mixture diluted in water with at least one of the following: lubricating agent, antistatic agent, cohesive agent and softener.

6. The method according to any one of the preceding claims, wherein the step of providing said textile product comprises the step of sorting said textile product from wool or recycled wool textile products.

7. The method according to any one of the preceding claims, wherein said sanitizing treatment step comprises a step of spraying said anti-contaminating mixture on said textile product.

8. The method according to any one of claims 1 to 6, wherein said sanitizing treatment step comprises a step of immersion of said textile product in said anti-contaminating mixture.

9. The method according to claim 8, wherein said immersion step further comprises a step of mechanical stirring of said textile product.

10. The method according to any one of the preceding claims, wherein said anti-contaminating mixture has a density at 20° of between 1.017 and 1.037 g/cm³, a pH value at 20° of between 3.0 and 4.0 and a refraction index at 20° of between 1.3380 and 1.3440.
